# EUROPEAN PATENT SPECIFICATION

(11) **EP 3 884 983 B1**
(45) Date of publication and mention of the grant of the patent: **16.11.2022**
(21) Application number: 20315047.9
(22) Date of filing: 25.03.2020
(51) Int. Cl.: A61M 16/00, A61M 16/20

(54) **MULTIFUNCTIONAL MANUAL ARTIFICIAL RESPIRATION BAG**
MULTIFUNKTIONALER BEUTEL ZUR MANUELLEN KÜNSTLICHEN BEATMUNG
SAC DE RESPIRATION ARTIFICIELLE MANUEL MULTIFONCTIONNEL

(43) Date of publication of application: 29.09.2021
(73) Proprietor: Air Liquide Medical Systems, 92160 Antony (FR)
(72) Inventor: Alberici, Luca, 25073 Bovezzo (IT); Richard, Jean-Christophe, 92160 Antony (FR); Zadra, Davide, 25073 Bovezzo (IT); Badat, Bilal, 92160 Antony (FR); Massaro, Paolo, 25073 Bovezzo (IT); Lesimple, Arnaud, 92160 Antony (FR)
(74) Representative: Air Liquide

(56) References cited:
- EP-A1- 3 556 415
- WO-A1-2005/035065
- WO-A1-2019/001751
- US-A1- 2012 012 111
- US-A1- 2019 366 029

## Description

The present invention relates to a multifunctional manual artificial respiration bag, such as manual resuscitation devices/systems, resuscitators or the like, that can be used for providing a respiratory gas to a person, such as a patient.

A manual resuscitation bag can be used for providing a respiratory gas, such as air, oxygen or a mixture thereof, to a person in need of a respiratory assistance, typically a patient. However, current manual artificial respiration bags are not useable for delivering gas to patients in various situations as they are generally dedicated for a given or specific task.

Thus, the most classical manual artificial respiration bags can only be used for providing a respiratory gas, such as air or an air/O₂ mixture, to a patient, during his/her transportation or transfer from a first place, such as an operation room or a radiotherapy room, to a second place, such as a recovery room or a bedroom, or vice versa. Examples of such bags are given by US-A-3,063,620, US-A-2017/0157348, US-A-4,501,271 or US-A-2,834,339.

However, such classical manual artificial respiration bags are not suitable for providing a respiratory gas to a person in state of cardiac arrest as they are not compatible or well-adapted with the cardiac massage that is done on the person.

This is why, some manual artificial respiration bags, called manual resuscitation bags, have been specifically designed for artificially ventilating a person in state of cardiac arrest, while thoracic compressions (TC), i.e. successive compressions and decompressions, are exerted by a rescuer on the thoracic cage of said person for restoring gas exchanges in the lungs and a blood circulation in the body and toward the organs, especially to the brain of the patient.

Examples of such manual resuscitation bags dedicated to "cardiac arrest" are given by WO-A-2019/001751 and WO-A-2019/001752. With such a manual resuscitation bag, it is possible to provide air or air/O₂ mixtures, even during chest compressions/decompressions. Other examples of manual resuscitation bags are described in WO-A-2017/096286, WO2015/041396, WO2005/035065 and EP-A-0743075.

A goal of the present invention is to provide an improved manual artificial respiration bag, namely a "multiuse" or "multifunctional" manual artificial respiration bag, that can be used in various situations for specific tasks induding transportation/transfer of patients from a first place to a second place, in an hospital, in the field or others locations, as well as with persons in state of cardiac arrest that undergo thoracic compressions.

A solution according to the present invention concerns a manual artificial respiration bag comprising:
- a deformable bag, i.e. flexible bag, comprising a gas inlet, a gas outlet and an inner volume for a respiratory gas,
- a downstream conduct element fluidly connected to the gas outlet of the deformable bag, and comprising an exhaust valve comprising an exhaust port, and
- a downstream one-way valve arranged into the downstream conduct element, said downstream one-way valve being configured for allowing a flow of respiratory gas to pass through said downstream one-way valve only toward the exhaust valve,
characterized in that it further comprises mobile port-closing means (i.e. a mobile port-closing device), actuatable by a user, cooperating with the exhaust port of the exhaust valve for at least partially closing said exhaust port thereby controlling the flow of respiratory gas passing through the exhaust port of the exhaust valve.

Depending on the embodiment, a manual artificial respiration bag according to the present invention can comprise of one or several of the following additional features:
- the mobile port-closing means are arranged on a mobile support-structure actuatable by the user.
- the mobile port-closing means at least partially close said exhaust port in response to an actuation of the mobile support-structure by the user, typically to a rotation or a translation of the support-structure by the user.
- the mobile port-closing means cooperate with the exhaust port of the exhaust valve for partially closing said exhaust port thereby limiting the flow of respiratory gas passing through said exhaust port of the exhaust valve, during the expiration phases of a patient, i.e. during the use of the manual artificial respiration bag.
- the support-structure carrying the port-closing means is rotatable, pivotable or translatable.
- the support-structure carrying the port-closing means is rotatable, i.e. configured for being mobile in rotation, when turned clockwise or counterclockwise by a user.
- the mobile port-closing means comprise a closing flap or wall, or the like.
- the closing flap or wall is carried by and/or integral with the mobile support-structure.
- the support-structure and the closing flap or wall are molded in one-piece.
- the mobile support-structure is coupled to (e.g. arranged on) the downstream conduct element.
- according to other embodiment, the port-closing means are arranged on the exhaust valve.
- the downstream conduct element comprises an inner passage or lumen for conveying gas.
- optionally, the manual artificial respiration bag further may comprise an over-pressure valve. In this embodiment, the downstream one-way valve may be arranged into the downstream conduct element (i.e. in its lumen) between the over-pressure valve and the exhaust valve.
- the downstream one-way valve comprises a valve-support arranged into the downstream conduct element, i.e. in its lumen, and a flexible valve body.
- the valve-support is rigid.
- the flexible valve body of the downstream one-way valve has an umbrella-shape or any other suitable form.
- the flexible valve body of the downstream one-way valve is made of a flexible material, such as an elastomer.
- the flexible valve body has an umbrella-shape comprising a disk-shape body and a rod element integral with said disk-shape body, preferably fixed at the center of said disk-shape body and projecting away from said disk-shape body.
- the valve-support comprises a support orifice.
- the rod element of the flexible valve body traverses the support orifice of the valve-support, i.e. the rod element is lodged into said support orifice
- the rotatable support-structure comprises at least one guiding-pin and the downstream conduct element comprises at least one guiding-groove, said guiding-pin being guided by and in said guiding-groove when the support-structure is moved/actuated, typically rotated, by the user, preferably (at least) two guiding-pins and (at least) two guiding-groove are provided.
- the exhaust port of the exhaust valve is more or less open depending on the position of the mobile closing flap or wall of the support-structure, i.e. the exhaust port is at least partially closed when the support-element is actuated by the user, e.g. rotated.
- the manual artificial respiration bag further comprises an upstream conduct element fluidly connected to the gas inlet of the deformable bag.
- the upstream conduct element comprises a PEP exhaust valve fluidly communicating with the ambient atmosphere for venting gas to the atmosphere when the gas pressure, into the upstream conduct element, exceeds a given pressure threshold.
- the upstream conduct element further comprises an air admission valve in fluid communication with the ambient atmosphere for allowing air to enter into the upstream conduct element.
- the upstream conduct element further comprises an oxygen port for fluidly connecting an oxygen source for providing oxygen.
- it further comprises an oxygen source fluidly connected to the oxygen port of the upstream conduct element, such as an oxygen-containing cylinder.
- the PEP exhaust valve arranged in the upstream conduct element comprises PEP-setting means for setting the desired pressure threshold.
- the upstream conduct element further comprises a reservoir port for fluidly connecting a flexible gas reservoir.
- it further comprises a flexible gas reservoir fluidly connected to the reservoir port of the upstream conduct element.
- the first conduct element comprises an oxygen port or entry arranged between the reservoir port for connecting the gas reservoir and the upstream one-way valve.
- the downstream conduct element further comprises an interface port for fluidly connecting a respiratory interface.
- it further comprises a respiratory interface fluidly connected the interface port of the downstream conduct element, preferably by means of a ball-head connector or the like.
- it further comprises an upstream one-way valve arranged into the upstream conduct element between the deformable bag and the PEP exhaust valve, said upstream one-way valve being configured for allowing a flow of respiratory gas to pass through said upstream one-way valve only toward the deformable bag.
- it further comprises a flow-restriction element arranged into the downstream conduct element.
- the PEP exhaust valve comprises a valve body and means for setting a desired pressure threshold comprising a rotatable member, actuatable by the user, arranged on the valve body and cooperating with pressure adjusting means arranged into the valve body.
- the opening pressure of the PEP exhaust valve is of between 0 cm H₂O and 30 cm H₂O, preferably of between 0 cm H₂O and 15 cm H₂O.
- the PEP exhaust valve comprises markings corresponding to several settable pressure values, in particular several settable pressure values of between 0 cm H₂O and 30 cm H₂O, for instance several pressure values comprising 0, 5 and 10 cmH₂O.
- the rotatable member of the PEP exhaust valve comprises an inner axially-projecting bulb cooperating with the pressure adjusting means arranged into the valve body.
- the pressure adjusting means arranged into the valve body comprise a piston head, a spring element and a valve seat cooperating with the piston head for adjusting the pressure threshold.
- the upstream conduct element comprises a inner passage or lumen.
- the valve body of the PEP exhaust valve is in fluid communication with the lumen of the first upstream conduct element.
- it further comprises a gas delivery conduct comprising the interface port and in fluid communication with the downstream conduct element for conveying at least part of the gas circulating into the gas conduct to a patient interface.
- the patient interface comprises of a respiratory mask or a tracheal cannula or probe.
- the overpressure valve arranged in the downstream conduct element is configured to vent to the atmosphere at least part of the gas present in the gas conduct, when the gas pressure in the downstream conduct element exceeds a given value.

Some embodiments according to the present invention are shown in the enclosed Figures, among which:
- Figures 1 and 2 represent side views of an embodiment of a manual artificial respiration bag according to the present invention,
- Figure 3 is an exploded scheme of a manual artificial respiration bag of according to the present invention,
- Figure 4 is an enlarged partial view of Figure 3,
- Figure 5 is an enlarged partial view of Figure 4,
- Figures 6 and 7 show the rotatable member of the resistance setting means in a first position, wherein the exhaust port is fully open (i.e. not closed),
- Figures 8 and 9 show the rotatable member of the resistance setting means in a second position, wherein the exhaust port is partially closed,
- Figures 10 and 11 are schemes of the downstream conduct element and the resistance setting means of a manual artificial respiration bag of according to the present invention, shown in the first and second positions, respectively, and
- Figure 12 is a cross-sectional view of the downstream conduct element and the resistance setting means of a manual artificial respiration bag of according to the present invention.

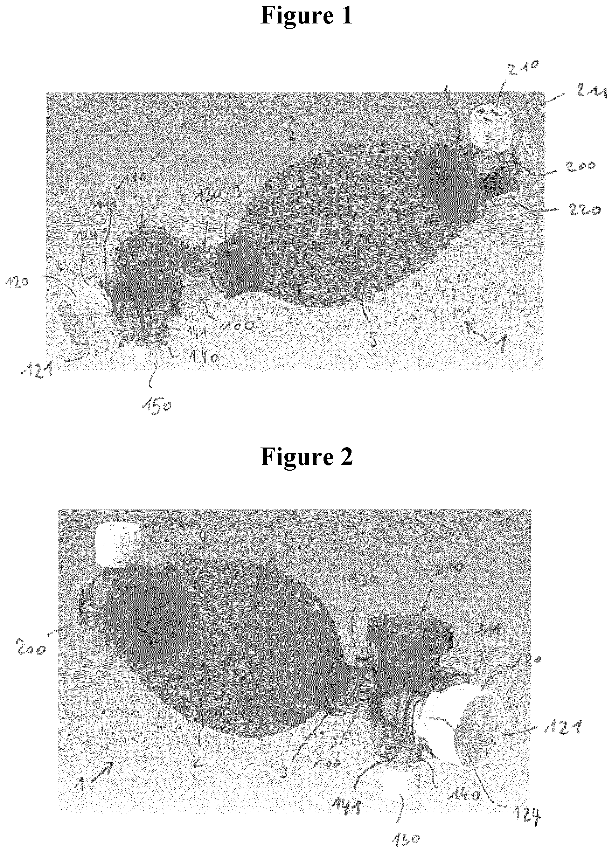

Figures 1 and 2 represent side views of an embodiment of a manual artificial respiration bag 1 according to the present invention. Said manual artificial respiration bag 1 generally comprises a deformable bag 2, i.e. flexible hollow bag, comprising an inner volume 5 for receiving a respiratory gas, such as air or a mixture of air and oxygen, on which a user, such as a rescuer (e.g: a physician), can exert a manual-pressure, i.e. that can be manually squeezed, for providing the respiratory gas to a patient, i.e. expelling the gas contained into the deformable bag 2 toward a patient in need thereof.

The deformable bag 2 further comprises a gas inlet 4 for introducing a respiratory gas into the inner volume 5 of the deformable bag 2 and a gas outlet 3 for delivering gas, while the deformable bag 2 is squeezed by a user. The deformable bag 2 is typically made of flexible material, typically a polymer material and has an inner volume 5 of preferably less than 2 L (i.e. when filled with water), for instance of about 1 L.

As shown in Figures 1 and 2, the manual artificial respiration bag 1 further comprises an upstream conduct element 200 fluidly connected to the gas inlet 4 of the deformable bag 2, and a downstream conduct element 100 fluidly connected to the gas outlet 3 of the deformable bag 2.

Both upstream and downstream conduct elements 200, 100 have a generally-tubular shape comprising a lumen for conveying the gas.

The upstream conduct element 200 comprises a PEP exhaust valve 210 fluidly communicating with the ambient atmosphere for venting gas (i.e. an over-pressure) to the atmosphere when the gas pressure, into the upstream conduct element 200, i.e. in its lumen, exceeds a given pressure threshold. In other words, the PEP exhaust valve 210 prevents gas overpressures in the deformable bag 2 and/or in the upstream conduct element 200 fluidly connected to the gas inlet 4 of the deformable bag 2.

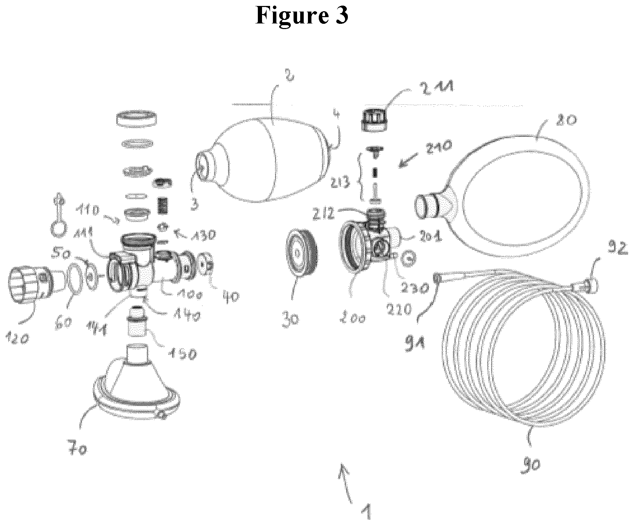

The upstream conduct element 200 further comprises an air admission valve 220 in fluid communication with the ambient atmosphere, for providing ambient air to the upstream conduct element 200, and preferably an oxygen port 230 (shown in Figure 3) for connecting an oxygen source thereto, such as an oxygen cylinder, for providing additional oxygen to the upstream conduct element 200 and thereby obtaining an oxygen/air mixture. The oxygen source is fluidly connected to the oxygen port 230 by means of a gas line 90 comprising gas connectors 91 or plugs, such as a flexible hose or the like, as shown in Figure 3.

Further, an upstream one-way valve 30 (shown in Figure 3) is arranged into the upstream conduct element 200 between the deformable bag 2 and the PEP exhaust valve 210, which is configured for allowing a flow of respiratory gas to pass through said upstream one-way valve 30 only toward the deformable bag 2, i.e. in the direction of the deformable bag 2.

The upstream conduct element 200 also comprises a reservoir port 201 for fluidly connecting a flexible gas reservoir 80, as shown in Figure 3.

Furthermore, the downstream conduct elements 100 may optionally comprise an over-pressure valve 130 (in some embodiments, such an over-pressure valve 130 may be not necessary) for venting to the atmosphere any over pressure in said downstream conduct elements 100, i.e. in its lumen.

The downstream conduct elements 100 further includes an exhaust valve 110 with an exhaust port 111 for venting to the atmosphere, the CO₂-enriched gases expired by the patient and/or coming out of the lungs of the patient.

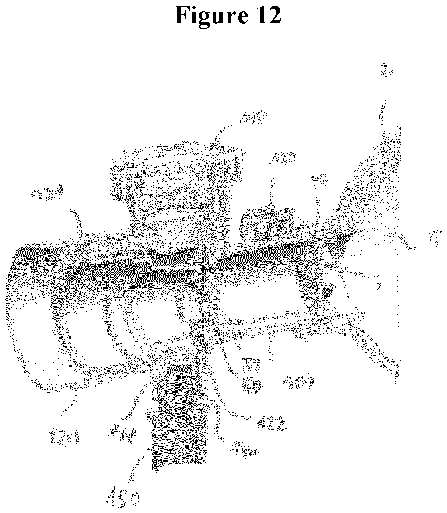

As shown in Figures 3-5 and 12, a downstream one-way valve 50, 55 is arranged into the downstream conduct element 100, i.e. into its lumen, for instance between the over-pressure valve 130 (when present) and the exhaust valve 110, and is configured for allowing a flow of respiratory gas to pass through said downstream one-way valve 50, 55 only toward the exhaust valve 110. In other words, the role of the downstream one-way valve 50, 55 is to control the way that the gas circulates into the downstream one-way valve 50, 55.

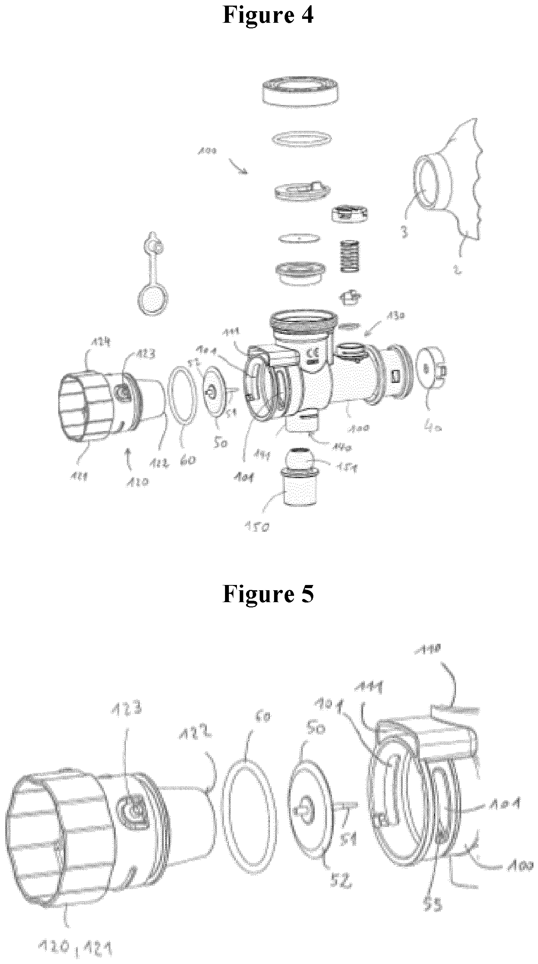

The respiratory gas, such as air or an air/O₂ mixture, flowing out of the deformable bag 2, when squeezed by a medical staff for instance, passes through the downstream conduct element 100 that is fluidly connected to the gas outlet 3 of the deformable bag 2, and is subsequently delivered to the patient's airways, by means of a respiratory interface 70, such as a facial mask, a laryngeal mask, an endotracheal tube or the like that fluidly connected to an interface port 140 of the downstream conduct element 100, as illustrated in Figures 3 and 4, preferably by means of a ball-head 151 hollow connector 150 or any other suitable tubular-connector. The interface port 140 of the downstream conduct element 100 can be arranged on a gas delivery conduct 141 branched to the downstream conduct element 100.

Aduantageously, the manual resuscitation bag 1 can comprise a handle (not shown) or the like for transporting it.

The PEP exhaust valve 210 comprises a rotatable member 211, such as a rotating knob or the like, actuatable by a user, namely a rescuer, a valve body 212 and means 213 for setting a desired pressure threshold including pressure adjusting means arranged into the valve body 212. Said pressure adjusting means 213 comprise a piston head, a spring element, such as a cylindrical spring, and a valve seat cooperating with the piston head for adjusting the pressure threshold as shown in Figure 3. The PEP exhaust valve 210 further comprises several markings corresponding to several settable pressure values, typically overpressure values of between 0 and 30 cm H₂O. In this aim, the rotatable member 211 further comprises an inner axially-projecting bulb (not visible), which cooperates with the pressure adjusting means 213 for adjusting the pressure threshold.

Further, the manual resuscitation bag 1 according to the invention can also comprise additional elements or features as explained below.

Thus, as shown in Figures 1-3, the upstream conduct element 200 further comprises an air admission valve 220 in fluid communication with the ambient atmosphere, an oxygen port 230 or entry for fluidly connecting a source of an oxygen-containing gas, such as or including a gas cylinder containing oxygen, which is delivered during insufflation phases. Such source of an oxygen-containing gas can be fluidly connected, via an oxygen line, such as a gas conduct, to the oxygen port 230 of the upstream conduct element 200. In this case, the flexible bag 2 can be filled with a mixture of oxygen and ambient air provided by the air admission valve 220 in fluid communication with the ambient atmosphere.

Furthermore, the downstream conduct element 100 fluidly connected to the gas outlet 3 of the deformable bag 2, comprises an exhaust valve 110 with an exhaust port 111, and a downstream one-way valve 50, 55 configured for allowing a flow of respiratory gas to pass through said downstream one-way valve 50, 55 only toward the exhaust valve 110, while circulating into the lumen of said downstream conduct element 100.

According to the present invention, the manual resuscitation bag 1 further comprises mobile port-closing means 124, actuatable by a user, cooperating with the exhaust port 111 of the exhaust valve 110 for at least partially closing said exhaust port 111 thereby controlling the flow of respiratory gas passing through the exhaust port 111 of the exhaust valve 110.

The mobile port-closing means 124 are arranged on a mobile support-structure 121 actuatable by the user.

Said mobile port-closing means are configured for at least partially closing the exhaust port 111 of the exhaust valve 110 in response to an actuation of the support-structure 121 by the user, typically to a rotation, pivoting or translation of the support-structure 121.

In the embodiment shown in the Figures, the mobile adjusting member 121 is rotatable, i.e. can be turned clockwise or counter-clockwise by the user, and is further coupled to the downstream conduct element 100. Nevertheless, other embodiments are possible, such as a pivoting or translating element, for instance a rigid curtain or the like, directly arranged on the exhaust valve 110.

More generally speaking, the mobile port-closing means 124 cooperate with the exhaust port 111 of the exhaust valve 110 for partially closing said exhaust port 111 thereby limiting the flow of respiratory gas passing through said exhaust port 111 of the exhaust valve 110, during the expiration phases of a patient. Of course, in opposite, if no flow limitation is desired, the port-closing means 124 are removed from the exhaust port 111 so that said exhaust port 111 is free, i.e. widely open, thereby letting a maximum flow of gas exiting through said exhaust port 111.

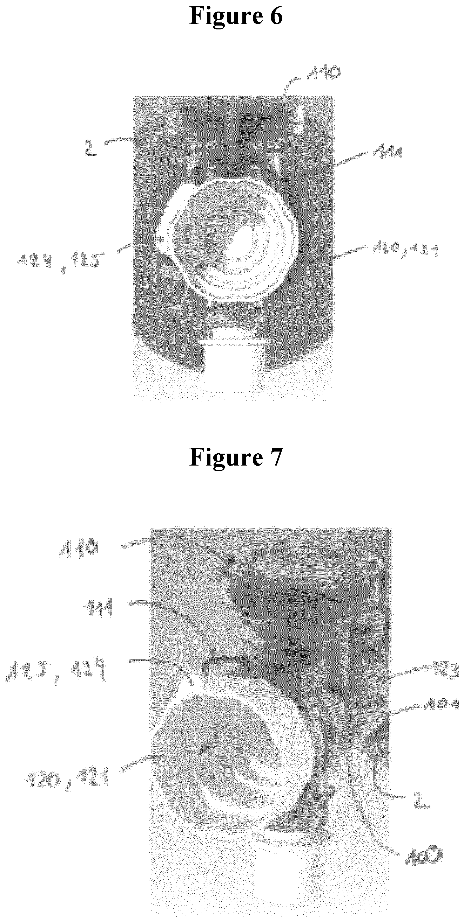

In the embodiment shown in the Figures, the mobile port-closing means 124 coupled to the downstream conduct element 100 comprise a closing flap or wall 125 (shown in Figure 6) that can partially occlude the exhaust port 111.

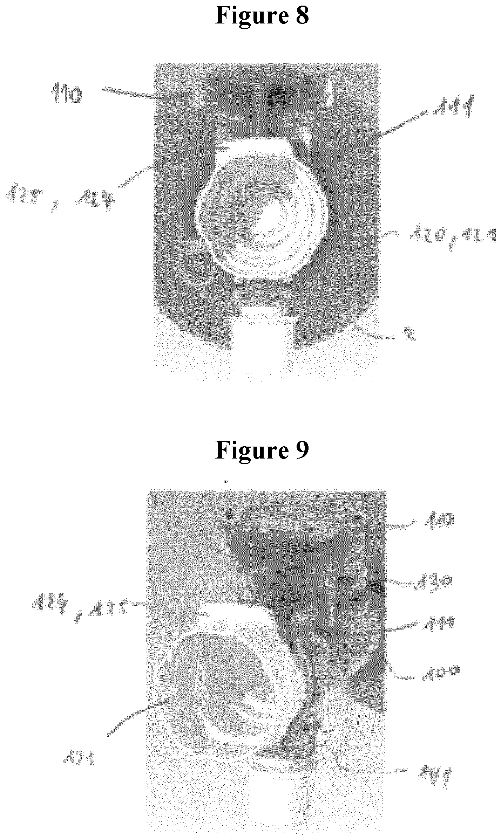

More precisely, the closing flap or wall 125 carried by the support-structure 121 that can be actuated by the user, typically rotated/turned (i.e. pivot), between several angular positions comprising :
- as shown in Fig. 6 and 7, a first angular position wherein it does not occlude/close the exhaust port 111 of the exhaust valve 110 so that a maximum flow of gas can be released to the atmosphere through said exhaust port 111, during the expiratory phases of a patient using such a bag 1,
- as shown in Fig. 8 and 9, a second angular position wherein it does occlude/close almost all of the exhaust port 111 of the exhaust valve 110, for instance about 90 or 95% of it (i.e. of its surface area), so that a minimum flow of gas can be released to the atmosphere through said exhaust port 111, during the expiratory phases of the patient, thereby increasing the flow resistance for the patient.

Of course other intermediary angular positions do also exist between said first angular position and second angular position, for more or less occluding/closing the exhaust port 111 of the exhaust valve 110.

Further, as shown in Figures 4 and 5, the downstream one-way valve 50, 55 comprises a valve support 55 arranged into the downstream conduct element 100 and a flexible valve body 50. The mobile adjusting member 121 acts directly or indirectly on the flexible valve body 50, when actuated by the user. The flexible valve body 50 is sandwiched between the mobile adjusting member 121 and the valve support 55. In the embodiment shown, the flexible valve body 50 of the downstream one-way valve 50, 55 has an umbrella-shape comprising a disk-shape body 52 and a rod element 51 integral with said disk-shape body 52, whereas the valve-support 55 comprises a support orifice, the rod element 51 of the flexible valve body 50 traversing said support orifice of the valve-support 55, i.e. the rod element 51 is positioned into the support orifice.

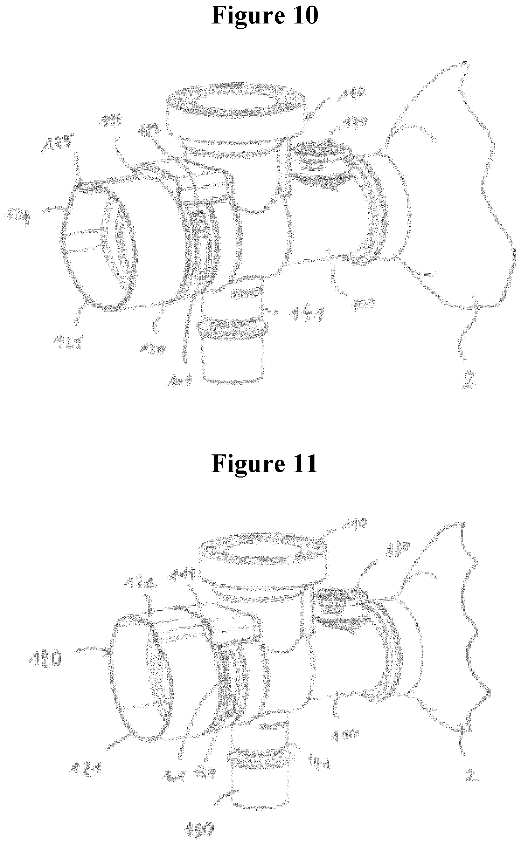

Preferably, as visible in Figures 4, 5, 10 and 11, the support-structure 121 further comprises one or several guiding-pins 123, whereas the downstream conduct element 100 comprises one or several guiding-grooves 101 that receive and guide the guiding-pins 123 and hence the motion of the support-structure 121, when the support-structure 121 is rotated by the user, for instance two guiding-pins 123 cooperating with two guiding-groove 101.

In the embodiment shown in the Figures, the support-structure 121 is a rotatable hand-wheel or the like having a general-tubular shape, and further comprising a annular part 126 that can be hand-gripped by the user for allowing said user to turn the hand-wheel clockwise or counterclockwise for closing or opening the exhaust port 111 of the exhaust valve 110 as above explained.

The closing flap 125 can be fixed to or carried by the outer wall of the rotatable support-structure 121. For instance, said closing flap or wall 125 can be made in one-piece, for instance molded in one-piece, with the support-structure 121. In other words, the support-structure 121 can be configured or designed to exhibit such a closing flap or wall 125, or the like.

According to another embodiment, the mobile port-closing means 124 can be fixed to another part of the manual resuscitation bag 1, in particular of the downstream conduct 100, i.e. not associated to or integral with the rotatable adjusting member 121.

Furthermore, as shown in Figures 3 and 4, it is also provided a flow-restriction element 40, such as a disk element carrying a calibrated orifice that is arranged into the downstream conduct element 100 for regulating the flow of gas delivered by the flexible reservoir 2.

The manual artificial respiration bag of the present can be used in various situations, for instance for resuscitating a person in state of cardiac arrest or the like, or for ventilation a person during transportation from one place to another place, in the field, in hospital, at the patient's home, in emergency vehicles or in any other place.

## Claims

1. Manual artificial respiration bag (1) comprising:
- a deformable bag (2) comprising a gas inlet (4), a gas outlet (3) and an inner volume (5) for a respiratory gas,
- a downstream conduct element (100) fluidly connected to the gas outlet (3) of the deformable bag (2), and comprising an exhaust valve (110) comprising an exhaust port (111), and
- a downstream one-way valve (50, 55) arranged into the downstream conduct element (100), said downstream one-way valve (50, 55) being configured for allowing a flow of respiratory gas to pass through said downstream one-way valve (50, 55) only toward the exhaust valve (110),
**characterized in that** it further comprises mobile port-closing means (124), actuatable by a user, cooperating with the exhaust port (111) of the exhaust valve (110) for at least partially closing said exhaust port (111) thereby controlling the flow of respiratory gas passing through the exhaust port (111) of the exhaust valve (110).

2. Manual artificial respiration bag according to the preceding claim, **characterized in that** the mobile port-closing means (124) are arranged on a mobile support-structure (121) actuatable by the user.

3. Manual artificial respiration bag according to claim 2, **characterized in that** the mobile port-closing means (124) at least partially close said exhaust port (111) in response to an actuation of the support-structure (121) by the user, typically to a rotation, pivoting or translation of the support-structure (121) by the user.

4. Manual artificial respiration bag according to any one of the preceding claims, **characterized in that** the mobile port-closing means (125) cooperate with the exhaust port (111) of the exhaust valve (110) for partially closing said exhaust port (111) thereby limiting the flow of respiratory gas passing through said exhaust port (111) of the exhaust valve (110), during the expiration phases of a patient.

5. Manual artificial respiration bag according to claim 3 or 4, **characterized in that** the mobile support-structure (121) carrying the mobile port-closing means (124) is rotatable, pivotable or translatable.

6. Manual artificial respiration bag according to any one of the preceding claims, **characterized in that** the mobile port-closing means (124) comprise a closing flap or wall (125).

7. Manual artificial respiration bag according to any one of the claims 2 to 6, **characterized in that** the mobile support-structure (121) carrying the port-closing means (124) is coupled to the downstream conduct element (100).

8. Manual artificial respiration bag according to claims 1-6, **characterized in that** the mobile port-closing means (124) are arranged on the exhaust valve (110).

9. Manual artificial respiration bag according to any one of the preceding claims, **characterized in that** the downstream one-way valve (50, 55) comprises a valve support (55) arranged into the downstream conduct element (100) and a flexible valve body (50).

10. Manual artificial respiration bag according to claim 9, **characterized in that**:
- the flexible valve body (50) has an umbrella-shape comprising a disk-shape body (52) and a rod element (51) integral with said disk-shape body (52), and
- the valve-support (55) comprises a support orifice, the rod element (51) of the flexible valve body (50) traversing said support orifice of the valve-support (55).

11. Manual artificial respiration bag according to any one of the preceding claims, **characterized in that** it further comprises an upstream conduct element (200) fluidly connected to the gas inlet (4) of the deformable bag (2), said upstream conduct element (200) comprising:
- a PEP exhaust valve (210) fluidly communicating with the ambient atmosphere for venting gas to the atmosphere when the gas pressure, into the upstream conduct element (200), exceeds a given pressure threshold, and
- an air admission valve (220) in fluid communication with the ambient atmosphere,
- and/or an oxygen port (230) for connecting an oxygen source.

12. Manual artificial respiration bag according to claim 11, **characterized in that** the PEP exhaust valve (210) arranged in the upstream conduct element (200) comprises PEP-setting means for setting the desired pressure threshold.

13. Manual artificial respiration bag according claims 11 or 12, **characterized in that**:
- the upstream conduct element (200) further comprises a reservoir port (201) for fluidly connecting a flexible gas reservoir (80), and
- the downstream conduct element (100) further comprises an interface port (140) for fluidly connecting a respiratory interface (70).

14. Manual artificial respiration bag according to any one of the claims 11 to 13, **characterized in that** it further comprises:
- an upstream one-way valve (30) arranged into the upstream conduct element (200) between the deformable bag (2) and the PEP exhaust valve (210), said upstream one-way valve (30) being configured for allowing a flow of respiratory gas to pass through said upstream one-way valve (30) only toward the deformable bag (2), and/or
- a flow-restriction element (40) arranged into the downstream conduct element (100).

15. Manual artificial respiration bag according to claim 13 or claim 14 when depending on claim 13, **characterized in that** it further comprises :
a flexible gas reservoir (80) fluidly connected to the reservoir port (201) of the upstream conduct element (200), and/or
- a respiratory interface (70) fluidly connected the interface port (140) of the downstream conduct element (100), preferably by means of a ball-head connector (150), and/or
- an oxygen source fluidly connected (90) to the oxygen port (230) of the upstream conduct element (200).

## Patentansprüche

1. Handbeatmungsbeutel (1), umfassend:
- einen verformbaren Beutel (2), umfassend einen Gaseinlass (4), einen Gasauslass (3) und ein Innenvolumen (5) für ein Atemgas,
- ein stromabwärtiges Leitungselement (100), in Fluidverbindung mit dem Gasauslass (3) des verformbaren Beutels (2) und ein Auslassventil (110) umfassend, das eine Auslassöffnung (111) umfasst, und
- ein stromabwärtiges Rückschlagventil (50, 55), angeordnet in dem stromabwärtigen Leitungselement (100), wobei das stromabwärtige Rückschlagventil (50, 55) dazu konfiguriert ist, nur einen Atemgasstrom durch das stromabwärtige Rückschlagventil (50, 55) zum Auslassventil (110) zuzulassen,
**dadurch gekennzeichnet, dass** es ferner bewegliche Öffnungsverschlussmittel (124) umfasst, die durch einen Benutzer betätigbar sind und mit der Auslassöffnung (111) des Auslassventils (110) zusammenwirken, um die Auslassöffnung (111) zumindest teilweise zu verschließen und dadurch die Strömung von Atemgas, das durch die Auslassöffnung (111) des Auslassventils (110) strömt, zu steuern.

2. Handbeatmungsbeutel nach dem vorhergehenden Anspruch, **dadurch gekennzeichnet, dass** die beweglichen Öffnungsverschlussmittel (124) auf einer beweglichen Stützstruktur (121) angeordnet sind, die durch den Benutzer betätigbar ist.

3. Handbeatmungsbeutel nach Anspruch 2, **dadurch gekennzeichnet, dass** die beweglichen Öffnungsverschlussmittel (124) die Auslassöffnung (111) als Reaktion auf eine Betätigung der Stützstruktur (121) durch den Benutzer, typischerweise eine Drehung, Schwenkung oder Verschiebung der Stützstruktur (121) durch den Benutzer, zumindest teilweise verschließen.

4. Handbeatmungsbeutel nach einem der vorhergehenden Ansprüche, **dadurch gekennzeichnet, dass** die beweglichen Öffnungsverschlussmittel (125) mit der Auslassöffnung (111) des Auslassventils (110) zusammenwirken, um die Auslassöffnung (111) zumindest teilweise zu verschließen und dadurch während der Ausatmungsphasen eines Patienten die Strömung von durch die Auslassöffnung (111) des Auslassventils (110) strömendem Atemgas einzuschränken.

5. Handbeatmungsbeutel nach Anspruch 3 oder 4, **dadurch gekennzeichnet, dass** die bewegliche Stützstruktur (121), die die beweglichen Öffnungsverschlussmittel (124) trägt, drehbar, schwenkbar oder verschiebbar ist.

6. Handbeatmungsbeutel nach einem der vorhergehenden Ansprüche, **dadurch gekennzeichnet, dass** die beweglichen Öffnungsverschlussmittel (124) eine Schließklappe oder -wand (125) umfassen.

7. Handbeatmungsbeutel nach einem der Ansprüche 2 bis 6, **dadurch gekennzeichnet, dass** die bewegliche Stützstruktur (121), die die Öffnungsverschlussmittel (124) trägt, mit dem stromabwärtigen Leitungselement (100) gekoppelt ist.

8. Handbeatmungsbeutel nach Anspruch 1-6, **dadurch gekennzeichnet, dass** die beweglichen Öffnungsverschlussmittel (124) an dem Auslassventil (110) angeordnet sind.

9. Handbeatmungsbeutel nach einem der vorhergehenden Ansprüche, **dadurch gekennzeichnet, dass** das stromabwärtige Rückschlagventil (50, 55) einen Ventilträger (55), der in dem stromabwärtigen Leitungselement (100) angeordnet ist, und einen flexiblen Ventilkörper (50) umfasst.

10. Handbeatmungsbeutel nach Anspruch 9, **dadurch gekennzeichnet, dass**:
- der flexible Ventilkörper (50) eine Regenschirmform aufweist, umfassend einen scheibenförmigen Körper (52) und ein Stabelement (51), das einstückig mit dem scheibenförmigen Körper (52) ausgebildet ist, und
- der Ventilträger (55) eine Trägeröffnung umfasst, wobei das Stabelement (51) des flexiblen Ventilkörpers (50) die Trägeröffnung des Ventilträgers (55) durchquert.

11. Handbeatmungsbeutel nach einem der vorhergehenden Ansprüche, **dadurch gekennzeichnet, dass** er ferner ein stromaufwärtiges Leitungselement (200) umfasst, in Fluidverbindung mit dem Gaseinlass (4) des verformbaren Beutels (2), wobei das stromaufwärtige Leitungselement (200) Folgendes umfasst:
- ein PEP-Auslassventil (210), in Fluidverbindung mit der umgebenden Atmosphäre, um Gas in die Atmosphäre abzulassen, wenn der Gasdruck in das stromaufwärtige Leitungselement (200) einen vorgegebenen Druckschwellenwert überschreitet, und
- ein Lufteinlassventil (220), in Fluidverbindung mit der umgebenden Atmosphäre,
- und/oder einen Sauerstoffanschluss (230) zum Anschließen einer Sauerstoffquelle.

12. Handbeatmungsbeutel nach Anspruch 11, **dadurch gekennzeichnet, dass** das in dem stromaufwärtigen Leitungselement (200) angeordnete PEP-Auslassventil (210) PEP-Einstellmittel zum Einstellen des gewünschten Druckschwellenwerts umfasst.

13. Handbeatmungsbeutel nach Anspruch 11 oder 12, **dadurch gekennzeichnet, dass**
- das stromaufwärtige Leitungselement (200) ferner eine Reservoiröffnung (201) zum fluidischen Verbinden eines flexiblen Gasreservoirs (80) umfasst, und
- das stromabwärtige Leitungselement (100) ferner eine Schnittstellenöffnung (140) zum fluidischen Verbinden einer Atemschnittstelle (70) umfasst.

14. Handbeatmungsbeutel nach einem der Ansprüche 11 bis 13, **dadurch gekennzeichnet, dass** er ferner Folgendes umfasst:
- ein stromaufwärtiges Rückschlagventil (30), das in dem stromaufwärtigen Leitungselement (200) zwischen dem verformbaren Beutel (2) und dem PEP-Auslassventil (210) angeordnet ist, wobei das stromaufwärtige Rückschlagventil (30) dazu konfiguriert ist, nur einen Atemgasstrom durch das stromaufwärtige Rückschlagventil (30) zum verformbaren Beutel (2) zuzulassen, und/oder
- ein Strömungsbegrenzungselement (40), das in dem stromabwärtigen Leitungselement (100) angeordnet ist.

15. Handbeatmungsbeutel nach Anspruch 13 oder nach Anspruch 14, wenn abhängig von Anspruch 13, **dadurch gekennzeichnet, dass** er ferner Folgendes umfasst:
- ein flexibles Gasreservoir (80), in Fluidverbindung mit der Reservoiröffnung (201) des stromaufwärtigen Leitungselements (200), und/oder
- eine Atmungsschnittstelle (70), in Fluidverbindung mit dem Schnittstellenanschluss (140) des stromabwärtigen Leitungselements (100), vorzugsweise mittels eines Kugelkopfverbinders (150), und/oder
- eine Sauerstoffquelle, in Fluidverbindung (90) mit dem Sauerstoffanschluss (230) des stromaufwärtigen Leitungselements (200).

## Revendications

1. Ballon de respiration artificielle manuelle (1) comprenant :
- un ballon déformable (2) comprenant une entrée de gaz (4), une sortie de gaz (3) et un volume interne (5) pour un gaz respiratoire,
- un élément de conduite aval (100) raccordé fluidiquement à la sortie de gaz (3) du ballon déformable (2), et comprenant une soupape d'échappement (110) comprenant un orifice d'échappement (111), et
- une valve unidirectionnelle aval (50, 55) agencée dans l'élément de conduite aval (100), ladite valve unidirectionnelle aval (50, 55) étant conçue pour permettre à un flux de gaz respiratoire de passer à travers ladite valve unidirectionnelle aval (50, 55) uniquement vers la soupape d'échappement (110),
**caractérisé en ce qu'**il comprend en outre des moyens de fermeture d'orifice mobiles (124), pouvant être actionnés par un utilisateur, coopérant avec l'orifice d'échappement (111) de la soupape d'échappement (110) pour fermer au moins partiellement ledit orifice d'échappement (111), contrôlant ainsi le flux de gaz respiratoire passant à travers l'orifice d'échappement (111) de la soupape d'échappement (110).

2. Ballon de respiration artificielle manuelle selon la revendication précédente, **caractérisé en ce que** les moyens de fermeture d'orifice mobiles (124) sont agencés sur une structure de support mobile (121) actionnable par l'utilisateur.

3. Ballon de respiration artificielle manuelle selon la revendication 2, **caractérisé en ce que** les moyens de fermeture d'orifice mobiles (124) ferment au moins partiellement ledit orifice d'échappement (111) en réponse à un actionnement de la structure de support (121) par l'utilisateur, en général à une rotation, un pivotement ou une translation de la structure de support (121) par l'utilisateur.

4. Ballon de respiration artificielle manuelle selon l'une quelconque des revendications précédentes, **caractérisé en ce que** les moyens de fermeture d'orifice mobiles (125) coopèrent avec l'orifice d'échappement (111) de la soupape d'échappement (110) pour fermer partiellement ledit orifice d'échappement (111) limitant ainsi le flux de gaz respiratoire passant à travers ledit orifice d'échappement (111) de la soupape d'échappement (110), pendant les phases d'expiration d'un patient.

5. Ballon de respiration artificielle manuelle selon la revendication 3 ou 4, **caractérisé en ce que** la structure de support mobile (121) portant les moyens de fermeture d'orifice mobiles (124) est rotative, pivotante ou déplaçable par translation.

6. Ballon de respiration artificielle manuelle selon l'une quelconque des revendications précédentes, **caractérisé en ce que** les moyens de fermeture d'orifice mobiles (124) comprennent un volet ou une paroi de fermeture (125).

7. Ballon de respiration artificielle manuelle selon l'une quelconque des revendications 2 à 6, **caractérisé en ce que** la structure de support mobile (121) portant les moyens de fermeture d'orifice (124) est accouplée à l'élément de conduite aval (100).

8. Ballon de respiration artificielle manuelle selon les revendications 1 à 6, **caractérisé en ce que** les moyens de fermeture d'orifice mobiles (124) sont agencés sur la soupape d'échappement (110).

9. Ballon de respiration artificielle manuelle selon l'une quelconque des revendications précédentes, **caractérisé en ce que** la valve unidirectionnelle aval (50, 55) comprend un support de valve (55) agencé dans l'élément de conduite aval (100) et un corps de valve flexible (50).

10. Ballon de respiration artificielle manuelle selon la revendication 9, **caractérisé en ce que** :
- le corps de valve flexible (50) a une forme de parapluie comprenant un corps en forme de disque (52) et un élément tige (51) solidaire dudit corps en forme de disque (52), et
- le support de valve (55) comprend un orifice de support, l'élément tige (51) du corps de valve flexible (50) traversant ledit orifice de support du support de valve (55).

11. Ballon de respiration artificielle manuelle selon l'une quelconque des revendications précédentes, **caractérisé en ce qu'**il comprend en outre un élément de conduite amont (200) raccordé fluidiquement à l'entrée de gaz (4) du ballon déformable (2), ledit élément de conduite amont (200) comprenant :
- une soupape d'échappement à PEP (210) en communication fluidique avec l'atmosphère ambiante pour évacuer le gaz vers l'atmosphère lorsque la pression du gaz, dans l'élément de conduite amont (200), dépasse un seuil de pression donné, et
- une soupape d'admission d'air (220) en communication fluidique avec l'atmosphère ambiante,
- et/ou un orifice d'oxygène (230) pour raccorder une source d'oxygène.

12. Ballon de respiration artificielle manuelle selon la revendication 11, **caractérisé en ce que** la soupape d'échappement à PEP (210) agencée dans l'élément de conduite amont (200) comprend des moyens de réglage de PEP pour régler le seuil de pression souhaité.

13. Ballon de respiration artificielle manuelle selon les revendications 11 ou 12, **caractérisé en ce que** :
- l'élément de conduite amont (200) comprend en outre un orifice de réservoir (201) pour le raccordement fluidique d'un réservoir de gaz flexible (80), et
- l'élément de conduite aval (100) comprend en outre un orifice d'interface (140) pour le raccordement fluidique d'une interface respiratoire (70).

14. Ballon de respiration artificielle manuelle selon l'une quelconque des revendications 11 à 13, **caractérisé en ce qu'**il comprend en outre :
- une valve unidirectionnelle amont (30) agencée dans l'élément de conduite amont (200) entre le ballon déformable (2) et la soupape d'échappement à PEP (210), ladite valve unidirectionnelle amont (30) étant conçue pour permettre à un flux de gaz respiratoire de traverser ladite valve unidirectionnelle amont (30) uniquement vers le ballon déformable (2), et/ou
- un élément de restriction de flux (40) agencé dans l'élément de conduite aval (100).

15. Ballon de respiration artificielle manuelle selon la revendication 13 ou la revendication 14 lorsqu'il dépend de la revendication 13, **caractérisé en ce qu'**il comprend en outre :
- un réservoir de gaz flexible (80) raccordé fluidiquement à l'orifice de réservoir (201) de l'élément de conduite amont (200), et/ou
- une interface respiratoire (70) reliée fluidiquement à l'orifice d'interface (140) de l'élément de conduite aval (100), de préférence au moyen d'un raccordement à tête sphérique (150), et/ou
- une source d'oxygène raccordée fluidiquement (90) à l'orifice d'oxygène (230) de l'élément de conduite amont (200).
